# EUROPEAN PATENT SPECIFICATION

(11) **EP 0 637 224 B1**
(45) Date of publication and mention of the grant of the patent: **30.12.1998**
(21) Application number: 93912244.6
(22) Date of filing: 14.04.1993
(51) Int. Cl.: A61B 17/11, A61F 2/06, A61F 2/24

(54) **VASCULAR IMPLANT SYSTEM**
VASKULARES IMPLANTATSYSTEM
SYSTEME D'IMPLANT VASCULAIRE

(30) Priority: 21.04.1992 US 871414
(43) Date of publication of application: 08.02.1995
(73) Proprietor: BAXTER INTERNATIONAL INC., Deerfield, IL 60015-4633 (US)
(72) Inventor: QUIJANO, R., C., Laguna Hills, CA 92653 (US); NASHEF, Aws, Newport Beach, CA 92646 (US)
(74) Representative: MacGregor, Gordon
(86) International application number: US9303517
(87) International publication number: WO9320757

(56) References cited:
- EP-A- 0 303 767
- WO-A-80/01460
- WO-A-89/02254
- WO-A-90/14804
- US-A- 2 127 903
- US-A- 2 453 056
- US-A- 3 254 650
- US-A- 3 254 651
- US-A- 4 086 665
- US-A- 4 247 292
- US-A- 4 340 977
- US-A- 4 352 358
- US-A- 4 728 328
- US-A- 5 089 014

## Description

### Field of the Invention

The present invention relates generally to bioprosthetic vascular implants and, more particularly, to stents and support/guide assemblies which are operative to (a) provide support for, (b) facilitate the implantation of, and (c) minimize thromboembolic complications resulting from artificial or bioprosthetic vascular implants.

### Background of the Invention

### i. Prosthetic Vascular Grafts Including Venous Valvular Implants

Modern vascular surgical procedures often involve the grafting of a tubular prosthetic implant of artificial or natural origin into an existing blood vessel for the purpose of replacing or bypassing a segment of diseased or damaged blood vessel. Many such procedures are accomplished by surgically removing the diseased or damaged segment of blood vessel, and subsequently replacing the removed segment of vessel with an appropriately sized tubular implant graft. The implant graft is typically held in place by anastomosing the ends of the implant graft to the opposing ends of the resected blood vessel.

In individuals who suffer from chronic venous valvular insufficiency, vascular grafting procedure have utilized to transplant functioning venous valves into the affected veins of the lower extremities. The transplantation of functioning venous valves in such individuals is therapeutically important as chronic incompetence or absence of venous valves into the veins of the lower extremities is known to give rise to numerous pathological consequences. For example, incompetence or absence of venous valves at the saphenofemoral or saphenopopliteal junctions may result in noncosmetic varices of the primary and/or secondary veins of the lower leg and ankle. Additionally, deep venous hypertension of the lower limb may occur. Such venous hypertension may result in lymphedema, aberrant pigmentation of the skin and, in severe cases, the formation of necrotizing lesions known as "venous ulcers".

Surgical transplantation of one or more functioning venous valves into a valve-deficient vein is a viable means of restoring venous valvular function to the valve deficient vein. The routine use of venous valve "transplant" procedures has heretofore been largely limited to autograft procedures. Such autograft procedures require the initial surgical excision of an autologous segment of viable vein (i.e. vein having a functioning venous valve therein) from one site within the patient's body, followed by subsequent transplantation of the harvested autograft to other veins wherein the venous valvular insufficiency has occurred. Such autograft transplant procedures are problematic because of (a) difficulties encountered in locating suitable segments of vein having viable venous valves therein and/or (b) the necessity of forming a separate incision or second surgery to harvest the venous valve autograft and/or (c) size mismatching of the harvested venous valve autograft relative to the implant site as may result in subsequent thromboembolic complications and failure of the implanted valve.

In view of the limitations and shortcomings of autograft venous valve transplantation procedures, it is desirable to develop artificial and/or preserved venous valve implants from cadaverous human or animal sources for subsequent transplantation into a human patient. The availability of artificial or bioprosthetic venous valve implants would eliminate the need for second-incision harvesting of homograft tissue and would enable the surgeon to select from an available range of graft sizes to obtain a graft which is specifically size-matched to the diameter of the resected blood vessel.

### ii. Presently Known Methods of Preparing Bioprosthetic Grafts

Various chemical tanning or "fixing" procedures have been used to preserve and prevent the breakdown of collagenous tissue grafts. Such "fixing" procedures generally involve the bathing or immersion of the collagenous graft tissue in a collagen cross-linking reagent. Examples of methods for preparing chemically cross-linked collagen or graft materials are found in U. S. Patent Nos. 2,900,644 (Rosenberg, et al.), 3,927,422 (Sawyer), 3,966,401 (Hancock, et al.), 3,974,526 (Dardik, et al.) , 4,239,492 (Holman, et al.) and 4,553,974 (Dewanjee).

Chemically fixed bioprosthetic heart valves and vascular grafts are commercially available. Examples of prosthetic heart valves constructed, at least in part, from chemically fixed biological tissue are described in U. S. Patent Nos. 4,372,734 (Lane) and 4,443,895 (Lane). Examples of bioprosthetic vascular grafts prepared from segments of mammalian blood vessel are found in U. S. Patent Nos. 4,671,797 (Varandecic) and 4,466,139 (Ketharanathan, et al.).

### iii. The Use of Stents to Support Bioprosthetic Tissue

Various rigid stent devices have heretofore been utilized to hold and support bioprosthetic implants, such as heart valves. Examples of stent devices for bioprosthetic heart valves are described in United States Patent Nos. 4,816,029 (Penny, III, et al.) and 4,851,000 (Gupta).

### iv. Thromboembolic Complications Known to Result from Turbulent Blood Flow Through Vascular Grafts

In the prior art, it has become recognized that abrupt variations in the lumenal diameter of a blood vessel, as may result from improper size matching of a vascular implant graft, may result in thromboembolic complications due to the resultant non-laminar or turbulent flow brought about the abrupt variation in lumenal diameter.

In particular, accurate size matching of vein grafts is difficult because certain peripheral veins normally undergo large amounts of dilation in performance of their normal physiological capacitance function. Thus, even if a vein graft is properly size matched at the time of the surgical implantation, subsequent dilation of the endogenous vein at a location to the non-dilating vein graft may give rise to abrupt variations in lumenal diameter of the blood vessel.

Accordingly, there remains a need in the art for improved vascular implant graft devices and techniques aimed at maximizing the biocompatibility and ease of use of such vascular implant grafts, while minimizing the potential for graft failure or other complications, such as immunoreactions to the implant graft material and/or thromboembolic complications resulting from turbulent blood flow through the implant graft.

### Summary of the Invention

The present invention overcomes some or all of the shortcomings of the prior art by providing an exovascular stent device according to Claim 1.

EP-A-0303767 discloses an anastomosis device comprising a short cylindrical body with end flanges. The device is engaged over the end of a blood vessel, with the end part of the vessel turned over the end flange and a clip engaged between the flanges over the cylindrical body. This arrangement avoids the use of sutures. Two such devices may be attached to opposite ends of a blood vessel segment, providing no support for the vessel between the two devices.

In this specification, the term "stent device" is used to mean a device, which not only holds a vessel segment, but also supports the segment between its ends.

### Brief Description of the Drawings

Figure 1 is a perspective view of an embodiment of an exovascular stent device positioned next to a bioprosthetic vein graft segment which has a venous valve located therein.
Figure 2 is a perspective view of the exovascular stent device.
Figure 3 is a perspective view of the exovascular stent device having a bioprosthetic vein graft operatively positioned therein.
Figure 4 is a longitudinal sectional view through line 4-4 of Figure 3.
Figure 5 is an end elevational view of the exovascular stent device shown in Figure 2.
Figure 6 is a perspective view of a tapered ring-support member usable in conjunction with the exovascular stent device.
Figure 7 is a perspective view of a gasket member usable in conjunction with the exovascular stent device and tapered ring-support device.
Figure 8 is a perspective view of an alternative gasket member usable in conjunction with the exovascular stent device and tapered ring-support member
Figure 9 is a perspective view of a vascular implant system comprising (a) an exovascular stent device; (b) two (2) dilation restrictor members and (c) two (2) gaskets, said system being shown in an in situ, operative position on a blood vessel.
Figure 10 is a longitudinal sectional view through line 10-10 of Figure 9.

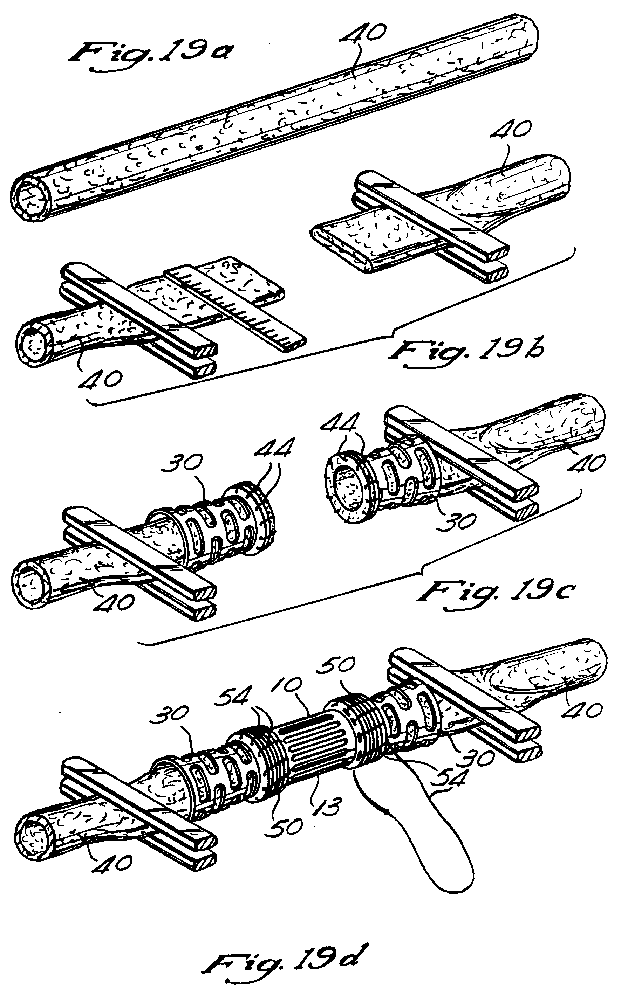

Figure 11 is a perspective view of a first alternative embodiment of a dilation restrictor member usable in conjunction with the exovascular stent device
Figure 12 is a perspective view of a second alternative embodiment of a dilation restrictor member usable in conjunction with the exovascular stent device.
Figure 13 is a perspective view of a first alternative embodiment of an exovascular stent device.
Figure 14 is a perspective view of a second alternative embodiment of an exovascular stent device.
Figure 15 is a perspective view of a modified single-piece embodiment of a vascular implant system of the present invention incorporating (a) an exovascular stent device component and (b) two (2) dilation restrictor member components.
Figure 16 is a longitudinal sectional view through line 16-16 of Figure 15.
Figure 17 is an exploded perspective view of a modified version of the vascular implant system shown in Figure 15, said modified version having tapered interfacing surfaces on adjacent components to facilitate alignment of the components.
Figure 18 is a longitudinal sectional view of the vascular implant system shown in Figure 17 when operatively positioned and mounted on an in situ blood vessel.
Figures 19a-19d are step-by-step schematic diagrams illustrating a method of implanting a prosthetic vascular graft utilizing a vascular implant system of the present invention.

### Detailed Descriptions of preferred Embodiments

The following detailed descriptions and the accompanying drawings are provided for the purpose of illustrating and describing certain presently preferred embodiments of the invention. The following detailed descriptions and drawings are not intended to limit the scope of the invention in any way.

### i. Exovascular Blood Vessel stent Device

In accordance with the invention there is provided an exovascular blood vessel stent device 10 which is useable to form a tubular implant prosthesis 13. The exovascular stent device 10 serves to hold and support a segment of tubular graft material which is a segment of blood vessel, vein or artery. In particular, the exovascular stent device 10 may be utilized in conjunction with a preserved segment of vein having a venous valve position therein. In such embodiment, the exovascular stent device 10 coupled with the preserved section of venous valve having the venous valve located therein results in the formation of a venous valve implant prosthesis.

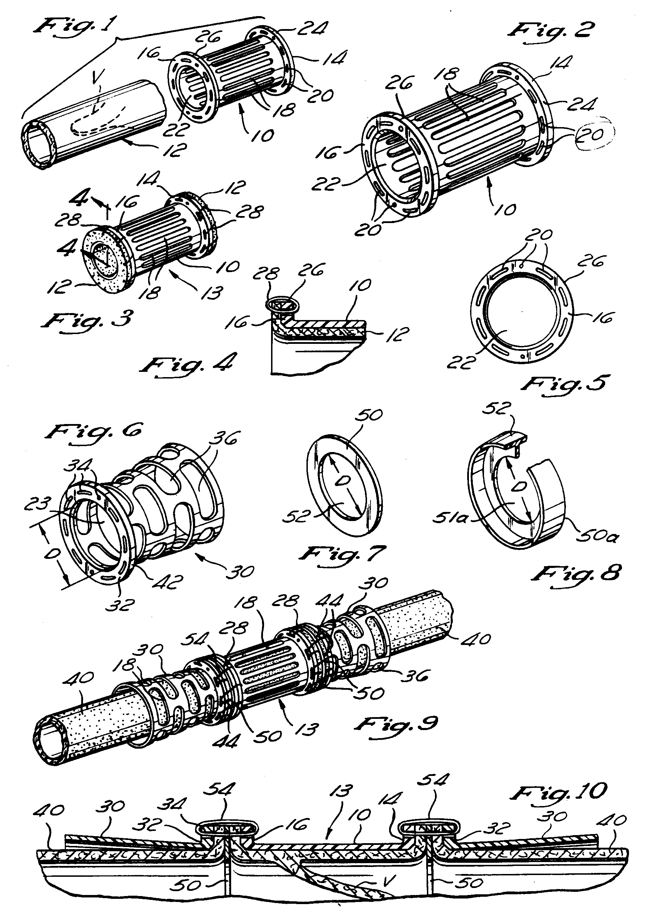

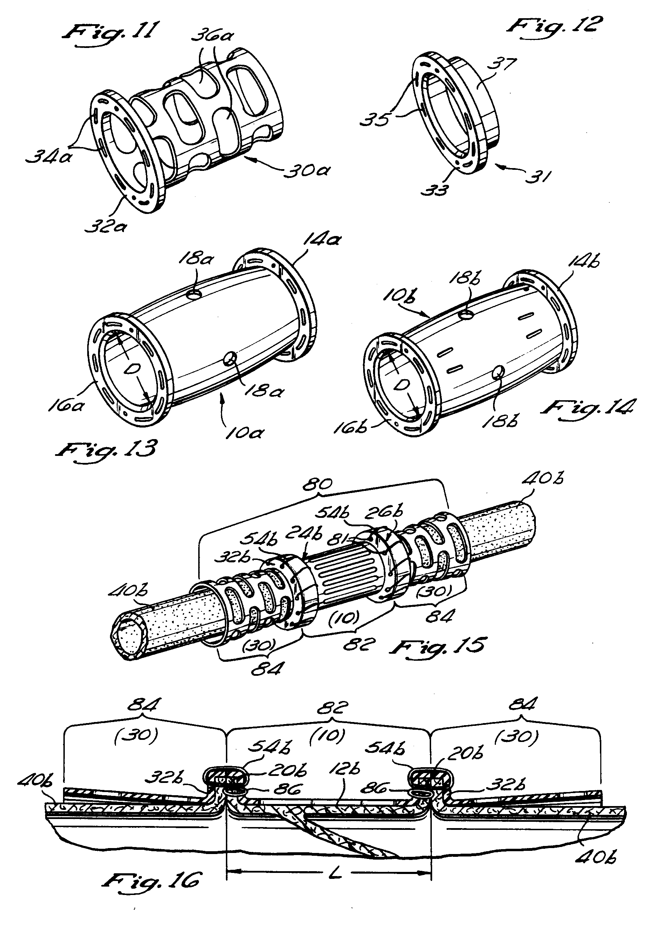

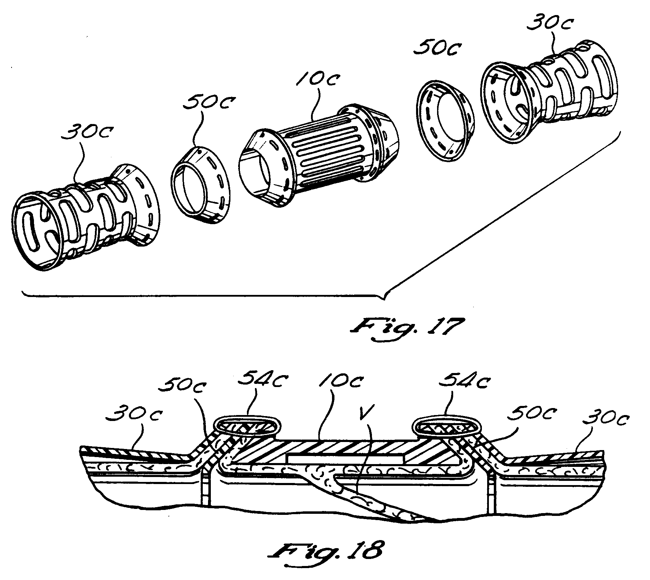

One embodiment of an exovascular stent device 10 is shown in Figures 1-5. Referring to Figures 1-5, the stent device 10 comprises a cylindrical body having a hollow inner bore 22 of round configuration extending longitudinally therethrough and having a plurality of fluid passage apertures 18, such as elongate slots (Fig. 2), formed around the body. The bore diameter may be 4 mm to 22 mm.

A first end flange 14 is formed on one end of the cylindrical stent body and a second end flange 16 is formed on the opposite end of the cylindrical stent body. Suture holes or apertures 20 are formed in end flanges 14, 16 to facilitate suturing of a bioprosthetic blood vessel segment 12 to the exovascular stent 10. The stent may be 2 to 6 cm long.

Initially, a segment of blood vessel 12, such as a segment of vein 12 having a venous valve (V) formed therein, is excised and removed from a cadaverous human or animal source. Excess tissue is removed from the segment of blood vessel 12 and the prepared segment of blood vessel 12 is thereafter immersed in or otherwise exposed to one or more chemical fixative or preservative solutions for a period of time sufficient to chemically fix or tan the collagenous matrix of the blood vessel segment 12, thereby forming a preserved bioprosthetic vascular graft.

Typically, the vein segment 10 is immersed in a chemical fixative solution known to cross-link collagen molecules for purposes of chemically "fixing" the collagenous network of the bioprosthetic vein graft. Examples of such chemical fixative solutions include formaldehyde, glutaraldehyde, dialdehyde starch, hexamethylene diisocyanate and certain polyepoxy compounds including glycol diglycidyl ether, polyol polyglycidyl ether, and dicarboxylic acid diglycidylester.

After the chemical fixation process has been completed, the "fixed" segment of blood vessel is inserted into the hollow bore 22 of the exovascular stent device 10 such that some portion of the vein segment 12 extends out of and protrudes beyond the opposite ends of the stent device 10. The protruding ends of the prosthetic vein segment 12 are then rolled back or splayed laterally such that they abut against the outer faces of the lateral end flanges 14, 16. Portions of the vein segment 12 which extend outboard of the outer edge of the flanges 14, 16 are then trimmed or cut away such that the ends of the vein segment 12 are substantially flush and even with the outer edges 24, 26 of the flanges 14, 16.

Sutures 28 are then passed through the ends of the vein segment 12 and through the suture apertures 20, thereby suturing the vein segment 12 to the exovascular stent device 10 to form a substantially unitary implant prosthesis which comprises 1.) the vein segment 12 and the surrounding stent 10. It is preferable that the suture apertures 20 be slightly elongate as shown, and sufficiently large to permit easy passage of a standard suture needle and suture material (e.g. 4-0 nylon) therethrough.

At the time of surgical implantation, the implant unit may be used in conjunction with one or two dilation restrictor members 30. Alternatively, the implant unit may be used with one or two anastomosis rims 31.

As shown in Fig.13, the stent 10a, having flanges 14a, 16a, may have an outwardly bowed body whose mid-point diameter is greater than the diameters at the ends. Apertures 18a may be provided.

### ii. Dilation Restrictor Members

There is provided a dilation restrictor member which functions to restrict the degree to which a blood vessel may dilate in a region immediately adjacent an existing suture line. The dilation restrictor member 30 is used in conjunction with the above-described exovascular stent device to form a complete vascular implant system.

The dilation restrictor member 30 comprises a generally cylindrical body having a flange member 32 formed on one end thereof. The cylindrical body of the dilation restrictor member 30 may be tapered such that the diameter of the bore of such cylindrical body is smaller at the end adjacent the flange 32 (e.g. 4 to 22 mm) than at the opposite end thereof (e.g. 5 to 24 mm). An example of such tapered configuration of the dilation restrictor member 30 is shown in Figure 6. In such tapered embodiment, it is desirable that the frusto-conical section of the restriction member 30 be configured such that its bore diameter gradually and continuously increases over the length of the bore, thereby providing a gradual taper against which the outer surface of the blood vessel may abut when the blood vessel undergoes dilation or diametric enlargement. A plurality of apertures 36 are formed in the body to permit passage of fluid therethrough.

The dilation restrictor member 30 serves two (2) functions. First, it operates as an appliance to (a) facilitate suturing of the implant prosthesis 13 into the desired blood vessel. Second, the dilation restrictor member operates to restrict dilation of the blood vessel 40 at regions immediately adjacent the points of anastomosis to the vascular implant prosthesis 13. By restricting or limiting the dilation of the blood vessel 40 at regions immediately adjacent the implant prosthesis 13, the dilation restrictor members 30 function to prevent or minimize variations in internal blood vessel diameter between the inner diameter of the implant prosthesis 13 and the inner diameter of the adjacent blood vessel 40. Such limitation helps to ensure laminar or nonturbulent flow of blood through the blood vessel 40 and implant prosthesis 13 without excessive turbulence.

In operation, the dilation restrictor member 30 is passed over the cut end of the blood vessel 40 such that the cut end of the blood vessel 40 protrudes slightly beyond the opening of the inner bore 23 of the dilation restrictor member 30 at the flange 32 end thereof. The end of the blood vessel 40 is then splayed outwardly such that the outer surface of the blood vessel 40 abut against the outboard face of the flange 32. The end of the blood vessel 40 is then cut or trimmed such that on dilation of the blood vessel 40 immediately adjacent the points of anastomosis to the implant prosthesis terminates flush with or substantially even with the outer periphery 42 of the flange 32. Sutures 44 are then passed through the end of the blood vessel 40 and the suture apertures 34 to secure the end of the blood vessel 40 to the flange 32.

The exposed luminal surface of blood vessel 40 which faces away from the outboard face of the flange 32 may then be placed in direct abutment with the exposed luminal surface of the prosthetic vein segment 12 which faces away from the outboard surface of adjacent lateral end flange 14 or 16 of the supporting stent device 10. A series of interrupted or noninterrupted sutures 50 may then be passed through the apertures 20, 34 and the interpositioned tissue of the blood vessel 40 and prosthetic vascular segment 12 to effect anastomosis of the prosthetic implant 13 to the blood vessel 40.

### iii. Optional Spacer Rings

An optional spacer ring or washer 50 may be interposed between the tissue of the blood vessel 40 and the tissue of the prosthetic vascular graft 12 to prevent the living blood vessel tissue 40 from coming in direct contact with the preserved tissue of the prosthetic vascular graft 12. The use of such spacer ring or washer 50 may minimize or prevent immunological reactions within the adjacent blood vessel 40 due to contact with the preserved tissue of the prosthetic vascular graft 12.

Such optional spacer ring or washer 50 may comprise a flat disc formed of biocompatible plastic such as Delrin™, acetyl resin (Dupont, Wilmington, Delaware 19898), Teflon™, or other suitable materials. The central aperture 52 of the spacer ring or washer 50 is preferably of the same inner diameter D as that of the flange end opening of the dilation restrictor member 30, and that of the end openings of the cylindrical bore 22 of the exovascular stent member 10. Such size matching of the inner diameters D of the adjacent portions of a.) the exovascular stent member 10, b.) the spacer ring washer 50, and c.) the central bore 23 of the dilation restrictor member 30 will prevent or minimize the likelihood of excessive turbulence or nonlaminar flow within the blood vessel due to excessive variations of inner diameter of the blood vessel, as may result if the implant components are not size matched. **iv. vascular Implant System and**

### Method of Use

The exovascular 10 may be coupled with one or more dilation restrictor members 30 to form a vascular implant system. Optionally, such vascular implant system may further incorporate spacer rings or washers 50. The individual exovascular stent 10, dilation restrictor member(s) 30 and optional spacer ring(s) or washer(s) 50 may be independently formed as separate components as shown in Figures 9, 10, 17 and 18 or, alternatively, may be formed as a single-piece system as shown in Figures 15 and 16.

In embodiments of the invention wherein the exovascular stent 10 dilation restrictor member (s) 30 and optional spacer ring(s) or washer(s) 50 are favored as separate components the dilation restrictor members 30 are positioned on the opposing cut ends of blood vessel 40 with each cut end of blood vessel 40 being splayed outwardly and affixed to the outer face of the dilation restrictor member flange. The bioprosthetic implant unit 13 incorporating the exovascular stent 10 is positioned therebetween. Spacer rings or washers 50 may be interposed between the opposing surfaces of the blood vessel 40 and the prosthetic vein segment 12 so as to prevent direct tissue-to-tissue contact therebetween. Full thickness sutures are then utilized to anastomose the components in end-to-end abutting relation, as shown in Figures 9 and 10. Notably, the sutures 54 pass through the flanges 14 or 16 and 32, through the adjacent out-turned tissue of the blood vessel and/or vascular implant 12 and through the optional washer or spacer 50. Such sutures 54 thus remain outside of the blood-transporting vessel lumen and do not come in contact with the flow of blood which normally passes through the vessel following the implant surgery.

### v. Anastomosis Rings

In applications where it is not desired to utilize a dilation restrictor member 30, a simple anastomosis ring 31, as shown in Figure 12, may be employed. Such anastomosis ring 31 comprises a rigid cylindrical rim 37 having a perpendicular flange 33 formed on one end thereof. suture apertures 35 are formed through the flange 33 as shown.

In operation, the rim 37 of the anastomosis ring 31 is passed over the outer surface of the cut end of blood vessel 40. The cut end of blood vessel is then splayed outwardly or rolled back such that the outer surface of the blood vessel abuts against the outboard surface of flange 33. The end of the blood vessel 40 is then cut or trimmed so as to terminate substantially flush with the outer periphery of flange 33.

Interrupted or uninterrupted sutures are passed through suture apertures 35 and the adjacent tissue of the blood vessel 40 to affix the anastomosis ring 31 to the cut end of the blood vessel 40 in the desired manner.

Thereafter, the luminal surface of the blood vessel 40 which faces away from the outboard surface of the flange 33 of anastomosis ring may be placed in direct abutment with the surface of prosthetic vein segment 12 which faces away from the outboard surface of the flange number 16 of the exovascular stent 10. Optionally, a spacer ring or washer 50 may be interposed between the opposing surfaces of the blood vessel 40 and the prosthetic vein segment 12, as described above with respect to the dilation restrictor member embodiment of the invention.

Interrupted or uninterrupted sutures 50 are then passed through the adjacent tissues of the blood vessel 40 and prosthetic vein segment 12, through the adjacent suture apertures 20 and 35 of the stent device 10 and anastomosis ring 31, respectively, and through any optionally interposed spacer ring or washer 50 so as to effect anastomotic coupling of the prosthetic implant 13 to the blood vessel 40.

In embodiments of the invention wherein the exovascular stent 10, dilation restrictor member(s) 30 and optional spacer ring(s) or washer(s) 50 are formed as a single piece system (80 Figures 15 and 16). The exovascular stent component 10 will comprise the mid-portion 82 of such single-piece system and will be formed of relatively rigid material such as acetyl resin (Delrin™, Dupont, Wilmington, Delaware 19898). The lateral end portions 84 of such single-piece system 80 comprise the dilation restrictor member(s) 30 and are formed of elastomeric material having greater elasticity than the relatively rigid mid-portion 82 of the single-piece system 80. Suture apertures 81 may be on the flange members 16b of the mid-portion 82 of the single-piece system 80 to permit passage of sutures 50A through the relatively rigid material of the mid-portion 82 of the system 80. On the otherhand, if the elastomeric material of the lateral end portions 84 of the single-piece system 80 is sufficiently flexible to be punctured by a suture needle, there need be no pre-cut suture apertures formed in the flange portion 32b of such relatively flexible lateral end portions 84 of the system 80.

Initially, a preserved segment of blood vessel 12b is positioned within the mid-region 82 of the single-piece system 80 such that the ends of the preserved segment of blood vessel 12b are splayed outwardly and positioned adjacent the end flanges 14b, 16b. The ends of the blood vessel segment 12b are affixed to the outboard surfaces of the end flanges 14b, 16b by way of an appropriate adhesive or by individual sutures 86. In embodiments where individual sutures 86 are employed, an additional set of suture passage apertures 26b may be formed in the end flanges 18b, 14b to accommodate passage of such sutures 86.

With the prosthetic segment of blood vessel 12d affixed within the mid-portion 82 of the single-pieced system 80, the entire system 80 may be sterilized and stored in an appropriate storage solution such as glutaraldehyde or dilute ethanol.

At the time of implantation, the system 80 having the prosthetic segment of blood vessel 24b mounted therein is rinsed and prepared for implantation. A section of blood vessel 40b is excised and removed. The removed section of blood vessel corresponds to the length L of the mid-region 82 of the single-piece system 80.

## Claims

1. An exovascular stent device (10) for supporting a segment of blood vessel between its ends, the stent device comprising:
an elongate tubular body have a hollow inner bore (22) extending longitudinally therethrough between first and second ends of the body, for receiving the segment of blood vessel in the bore;
first and second flange members (14,16) formed respectively about the first and second ends of the body; and
attachment means (20) on each flange member (14,16) adapting the flange members for attachment to opposite ends of the blood vessel segment received in said bore (22).

2. The exovascular stent device (10) of Claim 1, wherein said attachment means comprises a plurality of suture passage apertures (20) formed in each of said first and second flange members (14,16).

3. The exovascular stent device (10) of Claim 1 or 2, wherein a plurality of apertures (18) are formed in said tubular body to allow fluid to flow therethrough.

4. The exovascular stent device (10) of Claim 3, wherein the apertures comprise elongate slots (18).

5. The exovascular stent device (10) of any preceding claim, wherein said inner bore (22) is of round configuration.

6. The exovascular stent device of Claim 5, wherein said inner bore (22) has a diameter of 4 mm - 22 mm.

7. The exovascular stent device (10) of any preceding claim, wherein said stent device is 2 cm - 6 cm in length.

8. The exovascular stent device (10) of any preceding claim, wherein the elongate tubular body is outwardly bowed such that the cross-sectional dimension of said inner bore (22) at its approximate longitudinal midpoint is greater than its cross-sectional dimension of said inner bore (22) at either of said first and second ends thereof.

9. The exovascular stent device (10) of Claim 1, wherein the elongate tubular body comprises a plurality of suture apertures for suturing the segment of blood vessel thereto.

10. A vascular implant prosthesis, comprising:
an exovascular stent device (10) according to any preceding claim, and a preserved segment of blood vessel (12) positioned co-axially within the hollow bore (22) of said stent device (10) and attached to the first and second flanges (14,16) of said stent device (10) by said attachment means (20).

11. The vascular implant prosthesis of Claim 10, as appendant to Claim 2, including sutures (28) engaged in said suture holes (20) to attach the vessel (12) to the flange members (12,14).

12. The vascular implant prosthesis of Claim 10 or 11, wherein the first and second flange members (14,16) are perpendicular to an outer surface of the tubular body.

13. The vascular implant prosthesis of Claim 10, 11 or 12, wherein the segment of blood vessel (12) comprises a segment of vein.

14. The vascular implant prosthesis of Claim 13, wherein a venous valve is formed within said segment of vein.

15. The vascular implant prosthesis of Claim 14, wherein said venous valve has been preserved in an open position.

16. The vascular implant prosthesis of Claim 14, wherein said venous valve has been preserved in a closed position.

17. The vascular implant prosthesis of Claim 14, 15 or 16, wherein the vein segment has been preserved using a chemical fixative solution.

18. The vascular implant prosthesis of Claim 10, 11 or 12, wherein the segment of blood vessel (12) comprises a segment of artery.

19. The vascular implant prosthesis of any one of Claims 10 to 18 further comprising:
first and second members (30) respectively securable on cut end parts of a blood vessel (40) for holding the cut end parts, each member (30) adapted for coupling to one of the flange members (14,16).

20. The vascular implant prosthesis of Claim 19, wherein each first and second member (30) comprises an elongate tubular body having a hollow bore extending longitudinally therethrough and an outwardly extending flange (32) formed about one end of the tubular body for coupling to one of the flange members (14,16).

21. The vascular implant prosthesis of Claim 20, wherein the hollow bore of each first and second member (30) is tapered so as to have a smaller cross-sectional dimension at the flange member end thereof than at the other end.

22. A method of forming a valvular prosthesis, comprising the steps of: positioning a preserved segment of vein (12), having a venous valve therein,
coaxially within a hollow bore (22) of an exovascular stent device, the device having an elongate tubular body, the bore extending longitudinally therethrough between first and second ends of the body, with first and second flange members (14,16) formed respectively about the first and second ends of the body;
attaching a first end of the preserved segment of vein (12) to the first flange member (14); and
attaching a second end of the preserved segment of vein (12) to the second flange member (16);
whereby the stent device provides support for the length of the segment of vein (12) between the flange members (14,16).

23. The method of Claim 22, wherein the attaching steps comprise suturing the first and second ends of the segment of vein (12) to the respective flange members (14,16).

## Patentansprüche

1. Gefäßfremde Stenteinrichtung (10) zur Abstützung eines Blutgefäßsegments zwischen seinen Enden, wobei die Stenteinrichtung folgendes aufweist:
einen langgestreckten rohrförmigen Körper mit einer hohlen Innenbohrung (22), die zwischen einem ersten und einem zweiten Ende des Körpers in Längsrichtung durch ihn hindurch verläuft, um das Blutgefäßsegment in der Bohrung aufzunehmen;
einen ersten und einen zweiten Flanschteil (14, 16), die jeweils um das erste und das zweite Ende des Körpers herum gebildet sind; und
Befestigungsmittel (20) an jedem Flanschteil (14, 16), die die Flanschteile zur Befestigung an entgegengesetzten Enden des in der Bohrung (22) aufgenommenen Blutgefäßsegments geeignet machen.

2. Gefäßfremde Stenteinrichtung (10) nach Anspruch 1, wobei die Befestigungsmittel eine Vielzahl von Nahtmaterial-Durchtrittsöffnungen (20) aufweisen, die in jedem von dem ersten und dem zweiten Flanschteil (14, 16) gebildet sind.

3. Gefäßfremde Stenteinrichtung (10) nach Anspruch 1 oder 2, wobei eine Vielzahl von Öffnungen (18) in dem rohrförmigen Körper ausgebildet ist, um Fluiddurchfluß durch diese zuzulassen.

4. Gefäßfremde Stenteinrichtung (10) nach Anspruch 3, wobei die Öffnungen langgestreckte Schlitze (18) aufweisen.

5. Gefäßfremde Stenteinrichtung (10) nach einem der vorhergehenden Ansprüche, wobei die Innenbohrung (22) runde Konfiguration hat.

6. Gefäßfremde Stenteinrichtung nach Anspruch 5, wobei die Innenbohrung (22) einen Durchmesser von 4 mm bis 22 mm hat.

7. Gefäßfremde Stenteinrichtung (10) nach einem der vorhergehenden Ansprüche, wobei die Stenteinrichtung eine Länge von 2 cm bis 6 cm hat.

8. Gefäßfremde Stenteinrichtung (10) nach einem der vorhergehenden Ansprüche, wobei der langgestreckte rohrförmige Körper nach außen gewölbt ist, so daß die Querschnittsdimension der Innenbohrung (22) an ihrem ungefähren Längsmittelpunkt größer als die Querschnittdimension der Innenbohrung (22) an einem von ihrem ersten und zweiten Ende ist.

9. Gefäßfremde Stenteinrichtung (10) nach Anspruch 1, wobei der langgestreckte rohrförmige Körper eine Vielzahl von Nahtmaterial-Öffnungen aufweist, um das Blutgefäßsegment damit zu vernähen.

10. Gefäßimplantatprothese, die folgendes aufweist:
eine gefäßfremde Stenteinrichtung (10) nach einem der vorhergehenden Ansprüche und ein konserviertes Blutgefäßsegment (12), das koaxial innerhalb der hohlen Bohrung (22) der Stenteinrichtung (10) positioniert und an dem ersten und dem zweiten Flansch (14, 16) der Stenteinrichtung (10) durch die Befestigungsmittel (20) befestigt ist.

11. Gefäßimplantatprothese nach Anspruch 10 in Abhängigkeit von Anspruch 2, die Nahtmaterial (28) aufweist, das in die Nahtmaterialöffnungen (20) eingreift, um das Gefäß (12) an den Flanschteilen (12, 14) zu befestigen.

12. Gefäßimplantatprothese nach Anspruch 10 oder 11, wobei der erste und der zweite Flanschteil (14, 16) zu einer äußeren Oberfläche des rohrförmigen Körpers senkrecht sind.

13. Gefäßimplantatprothese nach Anspruch 10, 11 oder 12, wobei das Blutgefäßsegment (12) ein Venensegment aufweist.

14. Gefäßimplantatprothese nach Anspruch 13, wobei in dem Venensegment eine Venenklappe gebildet ist.

15. Gefäßimplantatprothese nach Anspruch 14, wobei die Venenklappe in einer offenen Position konserviert worden ist.

16. Gefäßimplantatprothese nach Anspruch 14, wobei die Venenklappe in einer geschlossenen Position konserviert worden ist.

17. Gefäßimplantatprothese nach Anspruch 14, 15 oder 16, wobei das Venensegment unter Anwendung einer chemischen Fixierlösung konserviert worden ist.

18. Gefäßimplantatprothese nach Anspruch 10, 11 oder 12, wobei das Blutgefäßsegment (12) ein Arteriensegment aufweist.

19. Gefäßimplantatprothese nach einem der Ansprüche 10 bis 18, das ferner folgendes aufweist:
ein erstes und ein zweites Element (30), die jeweils an abgeschnittenen Endteilen eines Blutgefäßes (40) befestigbar sind, um die abgeschnittenen Endteile zu halten, wobei jedes Element (30) zum Verbinden mit einem der Flanschteile (14, 16) ausgebildet ist.

20. Gefäßimplantatprothese nach Anspruch 19, wobei jedes erste und zweite Element (30) einen langgestreckten rohrförmigen Körper aufweist, der eine in Längsrichtung durch ihn verlaufende hohle Bohrung und einen nach außen verlaufenden Flansch (32) hat, der um ein Ende des rohrförmigen Körpers herum ausgebildet ist, um mit einem der Flanschteile (14, 16) zusammengefügt zu werden.

21. Gefäßimplantatprothese nach Anspruch 20, wobei die hohle Bohrung jedes ersten und zweiten Elements (30) so verjüngt ist, daß sie an ihrem flanschteilseitigen Ende eine kleinere Querschnittsdimension als an dem anderen Ende hat.

22. Verfahren zum Herstellen einer Herzklappenprothese, wobei das Verfahren die folgenden Schritte aufweist:
Positionieren eines konservierten Venensegments (12), das darin eine Venenklappe hat,
koaxial in einer hohlen Bohrung (22) einer gefäßfremden Stenteinrichtung, wobei die Einrichtung einen langgestreckten rohrförmigen Körper hat, die Bohrung in Längsrichtung zwischen einem ersten und einem zweiten Ende des Körpers durch diesen verläuft, wobei ein erstes und ein zweites Flanschelement (14, 16) um das erste bzw. das zweite Ende des Körpers herum gebildet sind;
Befestigen eines ersten Endes des konservierten Venensegments (12) an dem ersten Flanschteil (14); und
Befestigen eines zweiten Endes des konservierten Venensegments (12) an dem zweiten Flanschteil (16);
so daß die Stenteinrichtung eine Halterung für die Länge des Venensegments (12) zwischen den Flanschteilen (14, 16) bildet.

23. Verfahren nach Anspruch 22, wobei der Befestigungsschritt aufweist: Annähen des ersten und des zweiten Endes des Venensegments (12) an die jeweiligen Flanschteile (14, 16).

## Revendications

1. Dispositif de connexion exovasculaire (10) pour supporter un segment de vaisseau sanguin entre ses extrémités , le dispositif de connexion comprenant:
un corps tubulaire allongé ayant un passage intérieur creux (22) qui s'étend longitudinalement dans le corps, entre une première et une deuxième extrémités du corps, pour recevoir le segment de vaisseau sanguin dans ledit passage ;
une première et une deuxième brides (14,16) formées respectivement autour des première et deuxième extrémités du corps ; et
des moyens de fixation (20) sur chaque bride (14,16) pour permettre d'attacher les brides aux extrémités opposées du segment de vaisseau sanguin reçu dans ledit passage (22).

2. Dispositif de connexion exovasculaire (10) de la revendication 1, dans lequel lesdits moyens de fixation comprennent une pluralité de trous de passage de suture (20) formés dans chacune desdites première et deuxième brides (14,16).

3. Dispositif de connexion exovasculaire (10) selon la revendication 1 ou 2, dans lequel une pluralité d'ouvertures (18) sont ménagées dans ledit corps tubulaire pour permettre l'écoulement d'un fluide à travers celui-ci.

4. Dispositif de connexion exovasculaire (10) selon la revendication 3, dans lequel les ouvertures comprennent des lentes allongées (18).

5. Dispositif de conflexion exovasculaire (10) selon une quelconque des revendications précédentes, dans lequel ledit passage intérieur (22) est de configuration circulaire.

6. Dispositif de connexion exovasculaire selon la revendication 5, dans lequel ledit passage intérieur (22) a un diamètre de 4 mm à 22 mm.

7. Dispositif de connexion exovasculaire (10) selon une quelconque des revendications précédentes, dans lequel ledit dispositif de connexion a une longueur de 2 cm à 6 cm.

8. Dispositif de connexion exovasculaire (10) selon une quelconque des revendications précédentes, dans lequel le corps tubulaire allongé est renflé vers l'extérieur de sorte que la dimension de section transversale dudit passage intérieur (22),sensiblement à son point milieu longitudinal, est plus grande que la dimension de section transversale dudit passage intérieur (22) à l'une ou l'autre de sesdites première et deuxième extrémités.

9. Dispositif de connexion exovasculaire (10) selon la revendication 1, dans lequel le corps tubulaire allongé comporte une pluralité de trous de suture pour suturer le segment de vaisseau sanguin audit corps.

10. Prothèse d'implant vasculaire comprenant : un dispositif de connexion exovasculaire (10) selon une quelconque des revendications précédentes et un segment conservé de vaisseau sanguin (12) placé coaxialement dans le passage intérieur creux (22) dudit dispositif de connexion (10) et attaché aux première et deuxième brides (14,16) dudit dispositif de connexion (10) par lesdits moyens de fixation (20).

11. Prothèse d'implant vasculaire selon la revendication 10, lorsqu'elle dépend de la revendication 2, Incluant des sutures (28) engagées dans lesdits trous de suture (20) pour attacher le vaisseau (12) aux brides (14,16).

12. Prothèse d'implant vasculaire selon la revendication 10 ou 11, dans laquelle les première et deuxième brides (14,16) sont perpendiculaires à une surface extérieure du corps tubulaire.

13. Prothèse d'implant vasculaire selon la revendication 10, 11 ou 12, dans laquelle le segment de vaisseau sanguin (12) comprend un segment de veine.

14. Prothèse d'implant vasculaire selon la revendication 13, dans laquelle une valve veineuse est formée dans ledit segment de veine.

15. Prothèse d'implant vasculaire selon la revendication 14, dans laquelle ladite valve veineuse a été conservée dans une position ouverte.

16. Prothèse d'implant vasculaire selon la revendication 14, dans laquelle ladite valve veineuse a été conservée dans une position fermée.

17. Prothèse d'implant vasculaire selon la revendication 14, 15 ou 16, dans laquelle le segment de veine a été conservé au moyen d'une solution fixative chimique.

18. Prothèse d'implant vasculaire selon la revendication 10, 11 ou 12, dans laquelle le segment de vaisseau sanguin (12) comprend un segment d'artère.

19. Prothèse d'implant vasculaire selon une quelconque des revendications 10 à 18, comprenant en outre :
un premier et un deuxième éléments (30) qui peuvent être fixés respectivement sur des parties d'extrémité coupées d'un vaisseau sanguin (40) pour tenir les parties d'extrémité coupées, chaque élément (30) étant prévu pour couplage à une des brides (14,16).

20. Prothèse d'implant vasculaire selon la revendication 19, dans laquelle les premier et deuxième éléments (30) comprennent chacun un corps tubulaire allongé ayant un passage intérieur creux qui s'étend longitudinalement à travers ledit corps, et une bride s'étendant vers l'extérieur (32) formée autour d'une extrémité du corps tubulaire pour couplage à une des brides (14,16).

21. Prothèse d'implant vasculaire selon la revendication 20, dans laquelle le passage intérieur creux de chacun desdits premier et deuxième éléments (30) est conique de manière à avoir une dimension de section transversale plus petite à son extrémité du côté de la bride qu'à son autre extrémité.

22. Procédé de fabrication d'une prothèse valvulaire, comprenant les étapes de :
mise en place d'un segment conservé de veine (12), comportant une valve veineuse, coaxialement dans un passage intérieur creux (22) d'un dispositif de connexion exovasculaire, le dispositif ayant un corps tubulaire allongé, le passage intérieur s'étendant longitudinalement entre les première et deuxième extrémités du corps, et une première et une deuxième brides (14,16) étant formées respectivement autour des première et deuxième extrémités du corps ;
attache d'une première extrémité du segment conservé de veine (12) à la première bride (14) ; et
attache d'une deuxième extrémité du segment conservé de veine (12) à la deuxième bride (16) ;
de sorte que le dispositif de connexion procure un support pour la longueur du segment de veine (12) entre les brides (14,16).

23. Procédé selon la revendication 22, dans lequel les étapes d'attache comprennent la suture des première et deuxième extrémités du segment de veine (12) aux brides respectives (14,16).
